# EUROPEAN PATENT APPLICATION

(11) **EP 4 265 170 A1**
(43) Date of publication of application: **25.10.2023**
(21) Application number: 21921056.4
(22) Date of filing: 22.01.2021
(51) Int. Cl.: A61B 1/12

(54) **REPROCESSOR FOR MEDICAL DEVICE AND BOTTLE**

(71) Applicant: Olympus Medical Systems Corp., Tokyo 192-8507 (JP)
(72) Inventor: SHINTANI Shoko, Hachioji-shi, Tokyo 192-8507 (JP)
(74) Representative: Seemann & Partner Patentanwälte mbB
(86) International application number: PCT/JP2021/002315
(87) International publication number: WO 2022/157947

(57) **Abstract**

A reprocessor for medical equipment (1) to which a bottle (100) containing a liquid for reprocessing is set includes a sensor (68) configured to detect that the liquid is within a detection range. The sensor (68) is arranged such that, in a state where the bottle (100) is set to the reprocessor for medical equipment (1), a first inner wall surface (100a) of the bottle (100) is outside of the detection range and a stepped inner wall surface (100b) of the bottle (100) is inside of the detection range.

## Description

### Technical Field

The present invention relates to a reprocessor for medical equipment and to a bottle to be set to the reprocessor for medical equipment.

### Background Art

Techniques for detecting a remaining amount of a liquid in a bottle have been conventionally proposed.

For example, Japanese Patent Application Laid-Open Publication No. 2016-020224 describes an electric dispenser which discharges, using air supplied from an air pump, a content fluid such as hand soap or other cleaning agents or disinfectants. The electric dispenser includes a sensor unit in a dispenser body. For example, the sensor unit includes a contactless sensor such as a photoelectric sensor. The sensor unit is configured to detect a remaining amount of a content fluid stored in a container body based on a liquid level or a weight of the content fluid.

The reprocessor for medical equipment is an apparatus for reprocessing medical equipment such as an endoscope. A bottle containing a liquid for reprocessing (for example, cleaning/disinfecting) the medical equipment is set to the reprocessor for medical equipment. Reprocessors for medical equipment include a first type in which, every time one step of reprocessing of the medical equipment is performed, the liquid inside the bottle decreases by an amount used in one step, and a second type in which, once the bottle is set, an entirety of the liquid inside the bottle is introduced into the reprocessor for medical equipment to be used for a plurality of steps of reprocessing of the medical equipment. When the reprocessor for medical equipment is of the first type, a sensor is provided in order to detect a remaining amount of the liquid inside the bottle.

From the perspective of running cost, the sensor is preferably provided on the reprocessor for medical equipment as compared to being provided on the bottle to be replaced. As a sensor to be provided on the reprocessor for medical equipment, a sensor which does not come into contact with the liquid inside the bottle is generally used such as a capacitive sensor which detects a height of a liquid level inside the bottle in a contactless manner from a position in proximity to the bottle.

However, when sensors are provided on the reprocessor for medical equipment, variations occur among detected heights of the liquid level due to a difference in mounting positions of the sensors with respect to the reprocessor for medical equipment, a difference in housing positions when housing the bottle in the reprocessor for medical equipment, a difference in sensitivity among individual sensors, a difference in sensitivity among sensors in accordance with a type of the liquid contained in the bottle, and the like. As a result, cases may arise in which the number of performable steps of reprocessing of the medical equipment cannot be accurately assessed and, for example, even though a bottle contains an amount of liquid used in one step, the liquid ends up being discarded as residual liquid and is therefore wasted.

The present invention has been made in consideration of the circumstances described above and an object of the present invention is to provide a reprocessor for medical equipment capable of more accurately detecting a remaining amount of a liquid inside a bottle even when there is a variation in relative positions of the bottle containing the liquid and a sensor which detects a presence of the liquid and to provide a bottle to be set to the reprocessor for medical equipment.

### Disclosure of Invention

### Means for Solving the Problem

A reprocessor for medical equipment according to an aspect of the present invention is a reprocessor for medical equipment to which a bottle containing a liquid for reprocessing medical equipment is set, the reprocessor for medical equipment including a sensor configured to detect that the liquid is within a detection range, wherein the sensor is arranged such that, in a state where the bottle is set to the reprocessor for medical equipment, a first inner wall surface of the bottle arranged in a direction of gravitational force is outside of the detection range and a stepped inner wall surface connected to an upper side of the first inner wall surface at an angle intersecting the direction of gravitational force is inside the detection range.

A bottle according to an aspect of the present invention is a bottle which contains a liquid for reprocessing medical equipment and which is to be set to a reprocessor for medical equipment including a sensor configured to detect that the liquid is within a detection range, the bottle including: a first inner wall surface; and a stepped inner wall surface connected to the first inner wall surface, wherein in a state where the bottle is set to the reprocessor for medical equipment, the first inner wall surface is arranged in a direction of gravitational force and is outside of the detection range and the stepped inner wall surface is connected to an upper side of the first inner wall surface at an angle intersecting the direction of gravitational force and is inside the detection range.

A reprocessor for medical equipment according to an aspect of the present invention is a reprocessor for medical equipment to which a bottle containing a liquid for reprocessing medical equipment is set, the reprocessor for medical equipment including a sensor configured to detect that the liquid is within a detection range, wherein in a state where the bottle is set to the reprocessor for medical equipment, the sensor is configured to detect the liquid when a liquid level of the liquid is present above a step provided on a side surface of the bottle but configured not to detect the liquid when the liquid level of the liquid is present below the step.

### Brief Description of the Drawings

Fig. 1 is a perspective view showing an example of an external view of an endoscope reprocessor and an endoscope according to a first embodiment of the present invention;
Fig. 2 is a diagram showing an example of an internal configuration of the endoscope processor according to the first embodiment of the present invention;
Fig. 3 is a perspective view showing, from a rear surface side, a part of an internal configuration of a reprocessor body in a state where outer covering has been removed in the first embodiment of the present invention;
Fig. 4 is a diagram showing an enlargement of an arrangement portion of a bottle and a sensor shown in Fig. 3;
Fig. 5 is a diagram showing the sensor being separated from the bottle in a state where a tray has been drawn in the first embodiment of the present invention;
Fig. 6 is a diagram showing the sensor in proximity to the bottle in a state where the tray has been closed in the first embodiment of the present invention;
Fig. 7 is a diagram showing a situation where a stepped inner wall surface of the bottle is inside a detection range of the sensor and a liquid level of a medicinal solution is at a higher position than the stepped inner wall surface in the first embodiment of the present invention;
Fig. 8 is a diagram for describing how the sensor is switched from on to off when the liquid level of the medicinal solution drops to a height of the stepped inner wall surface of the bottle as long as the stepped inner wall surface is inside the detection range of the sensor and a first inner wall surface is outside of the detection range even when positions of the bottle and the sensor in a direction of gravitational force deviate from each other in the first embodiment of the present invention;
Fig. 9 is a perspective view showing, from the rear surface side, the bottle according to the first embodiment of the present invention;
Fig. 10 is a flow chart showing an action by a processor controlling a notification device to perform a notification based on a detection result of the sensor in the first embodiment of the present invention;
Fig. 11 is a chart showing a configuration example in which a stepped inner wall surface is an inner wall surface on a lower side of a convex wall provided at a predetermined height position of a bottle in comparison to other configuration examples in a second embodiment of the present invention; and
Fig. 12 is a diagram showing an arrangement of a sensor in a modification of the second embodiment of the present invention.

### Best Mode for Carrying Out the Invention

Hereinafter, embodiments of the present invention will be described with reference to the drawings. However, the present invention is not limited to the embodiments described below.

In the description of the drawings, same or corresponding elements are denoted by same reference signs when appropriate. In addition, note that drawings are schematic in nature and that a relationship among lengths of respective elements, a ratio among lengths of respective elements, and the like in a single drawing may differ from reality. Furthermore, even among a plurality of drawings, the drawings may include portions having a relationship or a ratio among lengths that differ from each other.

### [First embodiment]

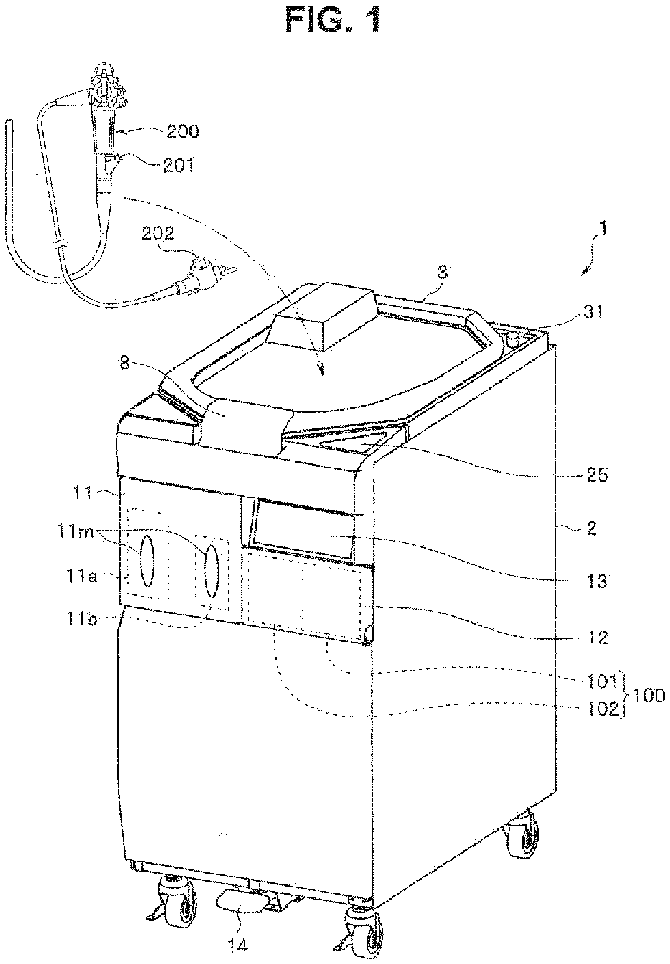

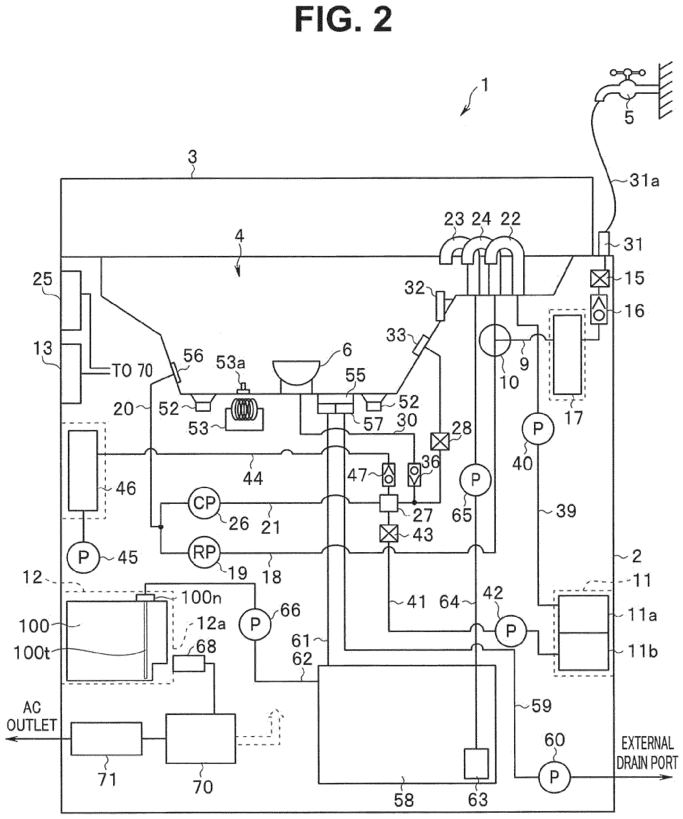

Figs. 1 to 10 represent a first embodiment of the present invention, in which Fig. 1 is a perspective view showing an example of an external view of an endoscope reprocessor 1 and an endoscope 200 and Fig. 2 is a diagram showing an example of an internal configuration of the endoscope reprocessor 1.

In the present embodiment, an example in which the medical equipment is the endoscope 200 and the reprocessor for medical equipment is the endoscope reprocessor 1 will be described. However, the medical equipment is not limited to the endoscope 200 and may be a treatment instrument, a medical appliance, a component of a medical appliance, and the like. Therefore, the reprocessor for medical equipment is not limited to the endoscope reprocessor 1.

The endoscope reprocessor 1 is an apparatus that reprocesses the endoscope 200 or an endoscope accessory. In this case, reprocessing may be rinsing with water, cleaning for removing contaminants such as organic substances, disinfection for neutralizing predetermined microorganisms, sterilization for eliminating or killing all microorganisms, or a combination of any two or more of these types of processing, and is not limited to specific processing.

As shown in Fig. 1, the endoscope reprocessor 1 includes a reprocessor body 2 and a top cover 3. The top cover 3 is connected to an upper part of the reprocessor body 2 using, for example, a hinge (not illustrated) and is openable and closable with respect to the reprocessor body 2.

In a state where the top cover 3 is closed on the reprocessor body 2, the reprocessor body 2 and the top cover 3 are fixed by, for example, a latch 8. The latch 8 is arranged at mutually-opposing positions of the reprocessor body 2 and the top cover 3.

A cleanser/alcohol tray 11 is arranged on a front surface and in an upper part of a left half of the reprocessor body 2 in Fig. 1. The front surface of the reprocessor body 2 is a surface with which an operator of the endoscope reprocessor 1 comes into proximity. An opposite side to the front surface of the reprocessor body 2 will be hereinafter referred to as a rear surface. The cleanser/alcohol tray 11 is drawable to the front (a direction of the front surface) of the reprocessor body 2.

The cleanser/alcohol tray 11 houses a cleanser tank 11a and an alcohol tank 11b. The cleanser tank 11a stores a cleaning agent which is used when cleaning the endoscope 200. The cleaning agent is a concentrated detergent which is diluted to a predetermined concentration with water. The water used to dilute the concentrated detergent is, for example, tap water filtered by a water supply filter 17 to be described later. The alcohol tank 1 1b stores alcohol which is used when drying the endoscope 200 after cleaning/disinfection. By drawing the cleanser/alcohol tray 11 to the front of the reprocessor body 2, the cleanser tank 11a can be refilled with the cleanser and the alcohol tank 1 1b can be refilled with alcohol.

The cleanser/alcohol tray 11 is provided with two windows 11m. The operator can check a remaining amount of the cleaning agent inside the cleanser tank 11a through one window 11m and check a remaining amount of alcohol inside the alcohol tank 11b through the other window 11m.

A tray 12 which houses the bottle 100 is provided on a front surface and in an upper part of a right half of the reprocessor body 2 in Fig. 1. The tray 12 is openable and closable with respect to the reprocessor body 2. By drawing the tray 12 to the front of the reprocessor body 2, the bottle 100 can be housed in the tray 12 or taken out from the tray 12. By closing the tray 12 housing the bottle 100, the bottle 100 is set to the endoscope reprocessor 1.

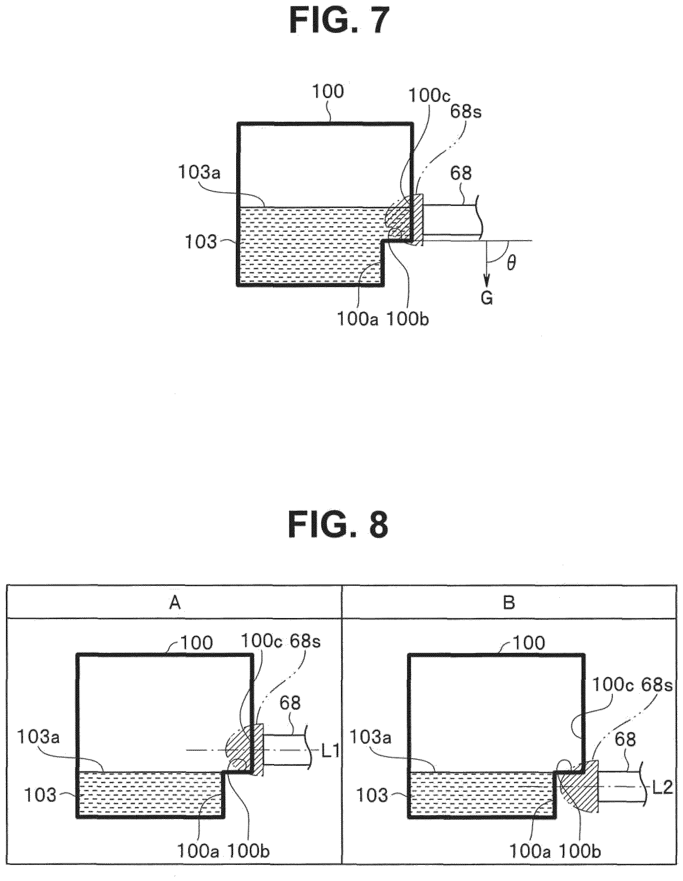

A medicinal solution 103 (refer to Fig. 7, Fig. 8, and the like) which is a liquid for disinfecting the endoscope 200 is present in the bottle 100. The medicinal solution 103 in an amount used in a plurality of steps of reprocessing (disinfection) of the endoscope 200 is present in the bottle 100 in an unused state. While an example in which the liquid in the bottle 100 is a liquid (the medicinal solution 103) for disinfecting the endoscope 200 will be described below, the liquid is not limited thereto. The liquid in the bottle 100 need only be a liquid for reprocessing the endoscope 200 or the like and may be a liquid for cleaning the endoscope 200 or the like or a liquid for assisting drying of the endoscope 200 or the like.

The bottle 100 includes a nozzle 100n for taking out the medicinal solution 103 inside the bottle 100. The endoscope reprocessor 1 according to the present embodiment is of a type in which the medicinal solution 103 inside the bottle 100 decreases by an amount used in one step every time one step of reprocessing of the endoscope 200 is performed.

For example, the bottle 100 includes a first bottle 101 containing a first medicinal solution and a second bottle 102 containing a second medicinal solution. Note that the bottle 100 may be constituted of one bottle or three or more bottles.

A sub operation panel 13 is arranged on the front surface of the reprocessor body 2 above the tray 12. A display device, instruction buttons, and the like are arranged on the sub operation panel 13. The display device includes a display panel or the like and displays, for example, a cleaning/disinfection time. For example, the instruction buttons include an operation button for heating the medicinal solution 103. The display device can also be used to notify a user and the sub operation panel 13 doubles as a notification device.

A main operation panel 25 is provided in a portion not covered by the top cover 3 such as a corner portion on the upper surface and a front surface side of the reprocessor body 2. Setting switches related to cleaning/disinfection of the reprocessor body 2 are arranged on the main operation panel 25. An example of the setting switches includes a start switch for starting a cleaning/disinfection operation and a selection switch for selecting a mode of cleaning/disinfection. The main operation panel 25 may include a display device such as a display panel and may double as a notification device for notifying the user.

A pedal switch 14 is arranged below the reprocessor body 2. When the operator steps on the pedal switch 14, the closed top cover 3 can be opened towards above the reprocessor body 2.

A water supply hose connecting port 31 is provided in a portion not covered by the top cover 3 such as a corner portion on the upper surface and a rear surface side of the reprocessor body 2. An end of a water supply hose 31a is connected to the water supply hose connecting port 31. Another end of the water supply hose 31a is connected to a water faucet 5 (refer to Fig. 2). Tap water from the water faucet 5 is supplied to the reprocessor body 2 via the water supply hose 31a and the water supply hose connecting port 31. A mesh filter for removing relatively large foreign objects in the tap water may be arranged in the water supply hose connecting port 31.

As shown in Fig. 2, the water supply hose connecting port 31 communicates with an end of a water supply conduit 9. Another end of the water supply conduit 9 is connected to a three-way solenoid valve 10. A water supply solenoid valve 15, a check valve 16, and a water supply filter 17 are provided midway along the water supply conduit 9 in order from a side of the water supply hose connecting port 31.

The water supply solenoid valve 15 is opened and closed by electric control and controls passage/cutoff of tap water. The check valve 16 is a non-return valve which allows the tap water inside the water supply conduit 9 to flow in a direction from the water supply solenoid valve 15 to the water supply filter 17 and prevents the tap water from flowing in an opposite direction. The water supply filter 17 is a filter which filters the tap water by removing foreign objects, bacteria, and the like in the tap water. For example, the water supply filter 17 is a cartridge-type filter which can be regularly replaced.

The three-way solenoid valve 10 internally includes a valve and is connected to one end of a liquid flow conduit 18. By operating the internal valve, the three-way solenoid valve 10 switches a communication destination of a water supply circulation nozzle 24 to one of the water supply conduit 9 and the liquid flow conduit 18. Specifically, by switching the internal valve of the three-way solenoid valve 10 to one side, the water supply circulation nozzle 24 is communicated with the water supply conduit 9 and, at the same time, cut off from the liquid flow conduit 18. In addition, by switching the internal valve of the three-way solenoid valve 10 to another side, the water supply circulation nozzle 24 is communicated with the liquid flow conduit 18 and, at the same time, cut off from the water supply conduit 9.

In addition, a liquid flow pump 19 is provided on a side of another end of the liquid flow conduit 18. The liquid flow pump 19 is a non-self-priming pump with superior liquid transfer capability which is capable of only transferring liquids.

The reprocessor body 2 includes a cleaning tank 4 in an upper part which becomes accessible when the top cover 3 is opened. The cleaning tank 4 houses the endoscope 200 and cleans and disinfects the endoscope 200 using a cleaning agent, the medicinal solution 103, or the like.

A circulation port 56 is arranged in the cleaning tank 4. The circulation port 56 is connected to one end of a circulation conduit 20. Another end of the circulation conduit 20 is branched in two. One of the two branches of the other end of the circulation conduit 20 is communicated with the other end of the liquid flow conduit 18. Another of the two branches of the other end of the circulation conduit 20 is communicated with one end of a channel conduit 21.

A channel pump 26, a channel block 27, and a channel solenoid valve 28 are provided midway along the channel conduit 21 in this order from a side of one end. The channel pump 26 is constituted of a self-priming pump and is capable of transferring both liquids and gases at higher pressure than a non-self-priming pump.

The channel conduit 21 is connected to one end of a case conduit 30 between the channel block 27 and the channel solenoid valve 28. Another end of the case conduit 30 is connected to a cleaning case 6. A relief valve 36 is provided midway along the case conduit 30.

Another end of the channel conduit 21 is communicated with an air/water feeding/forceps port 33. An air/water feeding channel 202, a forceps channel 201 (refer to Fig. 1), and the like of the endoscope 200 are connected using tubes to the air/water feeding/forceps port 33. In addition, although not illustrated, the other end of the channel conduit 21 is also communicated with a forceps raising port (not illustrated).

A water leak detection connector 32 is provided at a position higher than the air/water feeding/forceps port 33 in the cleaning tank 4. The water leak detection connector 32 is connected to a water leak detection pump (not illustrated) via a water leak detection conduit.

A cleanser nozzle 22 is connected to one end of a cleaning agent conduit 39. Another end of the cleaning agent conduit 39 is connected to the cleanser tank 11a. A cleanser pump 40 is provided midway along the cleaning agent conduit 39. The cleanser pump 40 is constituted of a high-pressure self-priming pump and pumps up a cleaning agent from the cleanser tank 11a to the cleaning tank 4.

The alcohol tank 1 1b is connected to one end of an alcohol conduit 41. Another end of the alcohol conduit 41 is connected to the channel block 27. The channel block 27 causes the alcohol conduit 41 to communicate with the channel conduit 21.

An alcohol supply pump 42 and a solenoid valve 43 are provided midway along the alcohol conduit 41. The alcohol supply pump 42 is constituted of a high-pressure self-priming pump and pumps up alcohol from the alcohol tank 1 1b to the cleaning tank 4.

One end of an air conduit 44 is further connected to the channel block 27. The channel block 27 causes the air conduit 44 to communicate with the channel conduit 21. Another end of the air conduit 44 is connected to an air pump 45. The air pump 45 is constituted of a self-priming pump capable of transferring gases and supplies air to the air conduit 44. A check valve 47 and an air filter 46 are provided midway along the air conduit 44. The air filter 46 is regularly replaced.

A drain port 55 is provided in a bottom part of the cleaning tank 4. A selector valve 57 is arranged in the drain port 55. The selector valve 57 is connected to one end of a drain conduit 59 and to one end of a medicinal solution recovery conduit 61. Another end of the medicinal solution recovery conduit 61 is connected to a medicinal solution tank 58.

A drain pump 60 is provided midway along the drain conduit 59. The drain pump 60 is constituted of a non-self-priming pump. Another end of the drain conduit 59 is connected to an external drain port via a drain hose (not illustrated).

By opening or closing the selector valve 57, the selector valve 57 is switched so that the drain port 55 and the drain conduit 59 are communicated with each other and, at the same time, the drain port 55 and the medicinal solution recovery conduit 61 are cut off from each other. Accordingly, a cleaning liquid/disinfectant or the like inside the cleaning tank 4 can be discharged from the external drain port.

By opening or closing the selector valve 57, the selector valve 57 is switched so that the drain port 55 and the medicinal solution recovery conduit 61 are communicated with each other and, at the same time, the drain port 55 and the drain conduit 59 are cut off from each other. Accordingly, the medicinal solution 103 inside the cleaning tank 4 can be collected in a medicinal solution tank 58.

The medicinal solution tank 58 is also connected to one end of a medicinal solution tube 62. Another end of the medicinal solution tube 62 is connected to the nozzle 100n of the bottle 100 housed in the tray 12. A suction tube 100t which is connected to the nozzle 100n and which suctions the medicinal solution 103 is provided inside the bottle 100. In addition, the bottle 100 may be provided with an air hole to be used when the medicinal solution 103 is suctioned. A suction pump 66 is provided midway along the medicinal solution tube 62. By operating the suction pump 66, the medicinal solution 103 is supplied from the bottle 100 to the medicinal solution tank 58. In this case, the first medicinal solution contained in the first bottle 101 and the second medicinal solution contained in the second bottle 102 may be mixed inside the medicinal solution tank 58. When the bottle 100 is constituted of three or more bottles, the medicinal solutions contained in the respective bottles may be mixed inside the medicinal solution tank 58.

In addition, one end portion of a medicinal solution conduit 64 is housed inside the medicinal solution tank 58. A suction filter 63 is provided at the one end of the medicinal solution conduit 64 which is housed inside the medicinal solution tank 58. Another end of the medicinal solution conduit 64 is connected to a medicinal solution nozzle 23. A medicinal solution pump 65 is provided midway along the medicinal solution conduit 64. The medicinal solution pump 65 is constituted of a high-pressure self-priming pump. When the medicinal solution pump 65 is operated, the medicinal solution 103 is pumped from the medicinal solution tank 58 and supplied to the cleaning tank 4 via the medicinal solution nozzle 23. Note that a configuration may be adopted in which the medicinal solution tube 62 is connected to the medicinal solution nozzle 23 and the medicinal solution 103 is directly supplied to the cleaning tank 4 from the bottle 100.

For example, two ultrasound transducers 52, a heater 53, and a temperature detection sensor 53a are arranged on a bottom surface of the cleaning tank 4. The ultrasound transducers 52 stir the liquid inside the cleaning tank 4 based on control by a processor 70 to be described later. The heater 53 heats the liquid inside the cleaning tank 4 based on control by the processor 70. The temperature detection sensor 53a detects a temperature of the liquid inside the cleaning tank 4 and outputs a detection result to the processor 70.

When the medicinal solution 103 is a disinfectant, there is a predetermined temperature (proper temperature) at which a disinfection effect can be maximized. Based on the temperature detected by the temperature detection sensor 53a, the processor 70 adjusts the temperature of the medicinal solution 103 inside the cleaning tank 4 to a proper temperature by controlling heating of the medicinal solution 103 by the heater 53. Due to the medicinal solution 103 having been adjusted to the proper temperature, the endoscope 200 housed in the cleaning tank 4 can be effectively disinfected. Furthermore, since the liquid stored in the cleaning tank 4 circulates inside the reprocessor body 2, each conduit inside the reprocessor body 2 can be effectively disinfected by the medicinal solution 103 having been adjusted to the proper temperature.

The endoscope reprocessor 1 includes a power supply 71 and the processor 70 which is electrically connected to the power supply 71. Power is supplied to the power supply 71 from an external AC outlet and the power supply 71 supplies power to each circuit in the endoscope reprocessor 1 including the processor 70.

The processor 70 includes a CPU, a memory, and the like and is configured to perform various kinds of processing related to the endoscope reprocessor 1 by causing the CPU to execute software read from the memory. The software stored in the memory is updatable. However, the processor 70 is not limited to this configuration and may be constituted of, for example, an electronic circuit which is configured to perform various kinds of processing. In addition, the processor 70 may be configured to include an integrated circuit such as an FPGA (Field Programmable Gate Array) including a circuit section configured to perform various kinds of processing.

The processor 70 receives various signals inputted from the main operation panel 25, the sub operation panel 13, the temperature detection sensor 53a, a sensor 68 to be described later, and the like and controls and drives the respective circuits in the endoscope reprocessor 1 including the main operation panel 25, the sub operation panel 13, and the respective pumps and the respective solenoid valves described above.

The software executed by the processor 70 includes, for example, a water supply conduit cleaning/disinfection program for cleaning/disinfecting the water supply conduit 9, an all conduit cleaning/disinfection program for cleaning/disinfecting insides of all conduits in the endoscope reprocessor 1, an endoscope cleaning/disinfection program for cleaning/disinfecting channels in the endoscope 200 via the air/water feeding/forceps port 33, and a medicinal solution remaining amount detection program for detecting and notifying of a remaining amount of the medicinal solution 103 inside the bottle 100.

When power of the processor 70 is turned on and, for example, the medicinal solution remaining amount detection program is executed, the processor 70 determines a remaining amount of the medicinal solution 103 inside the bottle 100 based on a detection result of the sensor 68, and when the processor 70 determines that the remaining amount has dropped to or below a predetermined amount, the processor 70 controls a notification device such as the main operation panel 25 or the sub operation panel 13 to notify the user of the remaining amount of the medicinal solution 103.

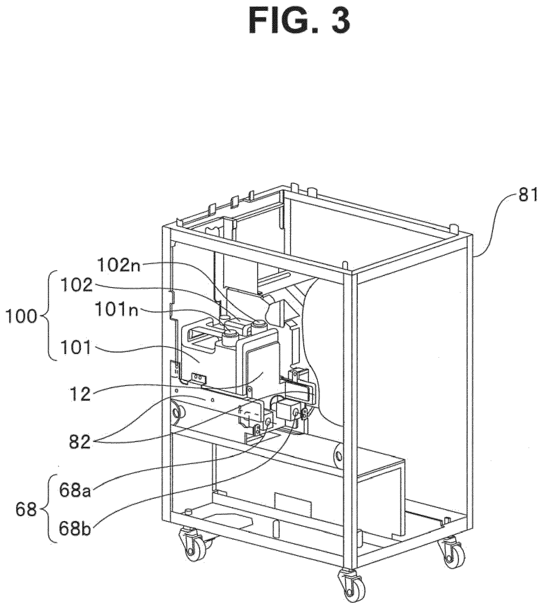

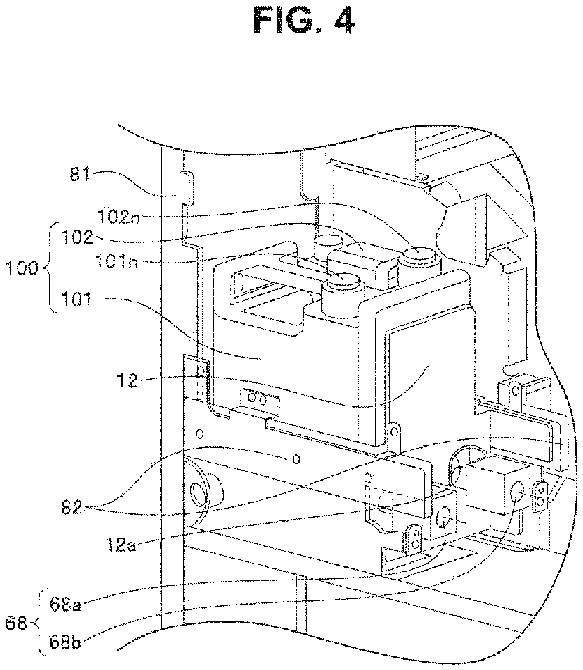

Fig. 3 is a perspective view showing, from a rear surface side, a part of an internal configuration of the reprocessor body 2 in a state where outer covering has been removed, and Fig. 4 is a diagram showing an enlargement of an arrangement portion of a bottle and a sensor shown in Fig. 3.

The reprocessor body 2 is provided with a chassis 81 inside an outer covering. The chassis 81 is provided with a pair of left and right rails 82. The rails 82 guide movements of the tray 12 in a horizontal direction and makes the tray 12 drawable. According to this structure, the tray 12 is drawn by being moved in a direction of a front surface of the reprocessor body 2 and closed by being moved in a direction of a rear surface of the reprocessor body 2.

As described earlier, for example, two bottles, namely, the first bottle 101 and the second bottle 102 are removably housed inside the tray 12. The first bottle 101 is provided with a nozzle 101n for taking out the medicinal solution that is present inside. The second bottle 102 is provided with a nozzle 102n for taking out the medicinal solution that is present inside.

A sensor hole 12a is provided on a surface on a rear surface side of the tray 12. The sensor 68 is provided at a position opposing the sensor hole 12a inside the reprocessor body 2. The sensor 68 detects that the medicinal solution 103 is within a detection range 68s (refer to Fig. 7, Fig. 8, and the like) without coming into contact with the medicinal solution 103. By arranging the sensor 68 as described below, the sensor 68 functions as a level sensor which detects, from a side surface of the bottle 100, a remaining amount of the medicinal solution 103 inside the bottle 100.

A specific example of the sensor 68 is a capacitive sensor. However, other contactless sensors such as an optical sensor may be used as the sensor 68. In addition, it goes without saying that a material suitable for constructing the bottle 100 changes depending on what kind of a sensor is used as the sensor 68. For example, when using a capacitive sensor as the sensor 68, the bottle 100 is desirably formed of a non-metallic material such as plastic. Alternatively, when a reflective optical sensor is used as the sensor 68, a material with optical transparency is used as the material that forms the bottle 100.

In the present embodiment, a first sensor 68a for detecting a presence or absence of the first medicinal solution inside the first bottle 101 and a second sensor 68b for detecting a presence or absence of the second medicinal solution inside the second bottle 102 are provided.

The first bottle 101 and the second bottle 102 may differ from each other in terms of shapes and sizes, and an amount of the first medicinal solution and an amount of the second medicinal solution which are used in one step of reprocessing generally differ from each other. For this reason, the first sensor 68a and the second sensor 68b may be arranged at different height positions.

In addition, since the first medicinal solution and the second medicinal solution are of different types, generally, sensitivities of the sensors also differ. For this reason, as the first sensor 68a and the second sensor 68b, sensors with different sensitivities may be used or sensors with different operating principles may be used.

Two sensor holes 12a including one for the first sensor 68a and one for the second sensor 68b may be provided or only one sensor hole 12a with a size common to the first sensor 68a and the second sensor 68b may be provided.

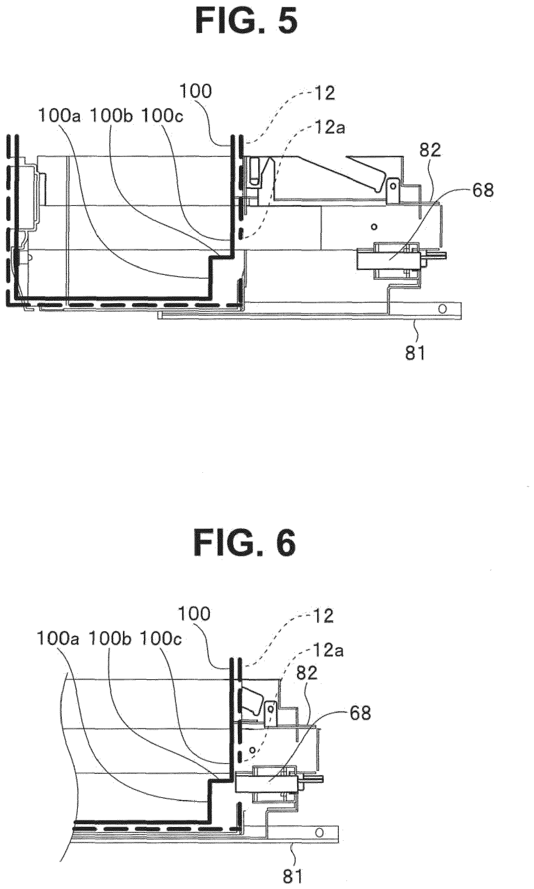

Fig. 5 is a diagram showing the sensor 68 being separated from the bottle 100 in a state where the tray 12 has been drawn, and Fig. 6 is a diagram showing the sensor 68 in proximity to the bottle 100 in a state where the tray 12 has been closed.

When the tray 12 is in a drawn state, as shown in Fig. 5, the sensor 68 is at a position separated from the bottle 100. At this point, the medicinal solution 103 inside the bottle 100 is not within the detection range 68s of the sensor 68.

On the other hand, when the tray 12 is closed, as shown in Fig. 6, the sensor 68 moves into proximity with the bottle 100 and the bottle 100 enters the detection range 68s of the sensor 68. At this point, the sensor 68 can detect the presence or absence of the medicinal solution 103 inside the bottle 100.

The detection of the presence or absence of the medicinal solution 103 inside the bottle 100 by the sensor 68 will be described in greater detail with additional reference to Fig. 7. Fig. 7 is a diagram showing a situation where a stepped inner wall surface 100b of the bottle 100 is inside the detection range 68s of the sensor 68 and a liquid level 103a of the medicinal solution 103 is at a higher position than the stepped inner wall surface 100b.

The bottle 100 includes a first inner wall surface 100a, the stepped inner wall surface 100b, and a second inner wall surface 100c. In this case, an inner wall surface refers to an inside surface of a wall portion with a predetermined thickness which constitutes the bottle 100 or, in other words, a surface in contact with the medicinal solution 103.

In a state where the bottle 100 is properly housed in the tray 12, the tray 12 is properly closed, and the bottle 100 is set to the endoscope reprocessor 1, the bottle 100 and the sensor 68 are arranged as follows.

The first inner wall surface 100a is arranged in a direction of gravitational force G. The second inner wall surface 100c is arranged in the direction of gravitational force G at a different position in the horizontal direction from the first inner wall surface 100a. Of the stepped inner wall surface 100b, a side of one end is connected to an upper side of the first inner wall surface 100a and a side of another end is connected to a lower side of the second inner wall surface 100c at an angle intersecting with the direction of gravitational force G. Therefore, the stepped inner wall surface 100b is a step provided on a side surface of the bottle 100. In addition, the stepped inner wall surface 100b is a surface which connects the first inner wall surface 100a arranged on a lower side and the second inner wall surface 100c arranged on an upper side.

The stepped inner wall surface 100b is provided at a position described below in the bottle 100 in a state of being set to the endoscope reprocessor 1. When n denotes an integer equal to or larger than 0, the stepped inner wall surface 100b is provided at a height position where the medicinal solution 103 in an amount that enables n or more and less than (n+1) number of steps of reprocessing of the endoscope 200 to be performed remains inside the bottle 100 at a time point where the medicinal solution 103 transitions from a state of being in contact with the stepped inner wall surface 100b to a state of not being in contact with the stepped inner wall surface 100b.

Directions along the first inner wall surface 100a and the second inner wall surface 100c need not be the same as the direction of gravitational force G as long as the first inner wall surface 100a and the second inner wall surface 100c are arranged in the direction of gravitational force G and may be slightly inclined. In other words, a direction perpendicular to the first inner wall surface 100a may or may not be orthogonal to the direction of gravitational force G. In a similar manner, a direction perpendicular to the second inner wall surface 100c may or may not be orthogonal to the direction of gravitational force G. Furthermore, the first inner wall surface 100a and the second inner wall surface 100c are not limited to being flat surfaces and may be curved surfaces as long as the first inner wall surface 100a and the second inner wall surface 100c are arranged in the direction of gravitational force G.

When an angle orthogonal to the direction of gravitational force G is selected as an angle at which the stepped inner wall surface 100b intersects with the direction of gravitational force G, there is a possibility that, even when the liquid level 103a drops below the stepped inner wall surface 100b, droplets of the medicinal solution 103 remain on the stepped inner wall surface 100b and the sensor 68 detects a presence of the medicinal solution 103. In consideration thereof, the stepped inner wall surface 100b favorably intersects with the direction of gravitational force G at an angle θ close to orthogonal so that a declivity is formed from a connection portion with the second inner wall surface 100c toward a connection portion with the first inner wall surface 100a. At this point, the angle θ shown in Fig. 7 is θ > 90°.

The sensor 68 is arranged so that the first inner wall surface 100a is outside the detection range 68s and the stepped inner wall surface 100b is inside the detection range 68s. More specifically, the sensor 68 does not detect the medicinal solution 103 regardless of whether the medicinal solution 103 is in contact with or not in contact with the first inner wall surface 100a. In addition, when the medicinal solution 103 is in contact with the stepped inner wall surface 100b inside the detection range 68s, the sensor 68 detects the medicinal solution 103. On the other hand, when the medicinal solution 103 is not in contact with the stepped inner wall surface 100b, the sensor 68 does not detect the medicinal solution 103.

Hereinafter, a description will be given as appropriate based on an assumption that the sensor 68 is turned on when detecting the medicinal solution 103 and the sensor 68 is turned off when not detecting the medicinal solution 103. However, it goes without saying that a configuration may be adopted in which a correspondence relationship between detections results and on/off states is reversed such that the sensor 68 is turned off when detecting the medicinal solution 103 and the sensor 68 is turned on when not detecting the medicinal solution 103.

Fig. 8 is a diagram for describing how the sensor 68 is switched from on to off when the liquid level 103a of the medicinal solution 103 drops to a height of the stepped inner wall surface 100b of the bottle 100 as long as the stepped inner wall surface 100b is inside the detection range 68s of the sensor 68 and the first inner wall surface 100a is outside of the detection range 68s even when positions of the bottle 100 and the sensor 68 in the direction of gravitational force G deviate from each other.

Field A and field B in Fig. 8 show states where the bottle 100 is set to the endoscope reprocessor 1 and, in both fields A and B, the first inner wall surface 100a is outside of the detection range 68s of the sensor 68.

Field A in Fig. 8 represents an arrangement example in which a height L1 of a central axis of the sensor 68 is higher than the stepped inner wall surface 100b and at least a part of the stepped inner wall surface 100b is included in a lower side in the detection range 68s of the sensor 68.

In addition, field B in Fig. 8 represents an arrangement example in which a height L2 of the central axis of the sensor 68 is lower than the stepped inner wall surface 100b and at least a part of the stepped inner wall surface 100b is included in an upper side in the detection range 68s of the sensor 68.

In both arrangements of fields A and B in Fig. 8, the sensor 68 is turned on and detects the medicinal solution 103 when the liquid level 103a of the medicinal solution 103 is present above the stepped inner wall surface 100b being a step provided on a side surface of the bottle 100 but the sensor 68 is turned off and does not detect the medicinal solution 103 when the liquid level 103a of the medicinal solution 103 is present below the stepped inner wall surface 100b being a step.

Therefore, when the stepped inner wall surface 100b is inside the detection range 68s and the first inner wall surface 100a is outside of the detection range 68s, the sensor 68 transitions from on to off at a time point where a remaining amount becomes approximately the same even if positions of the sensor 68 and the bottle 100 in the direction of gravitational force G deviate from each other. Accordingly, even if positions of the sensor 68 and the bottle 100 in the direction of gravitational force G deviate from each other, a remaining amount of the medicinal solution 103 inside the bottle 100 at a time point where the sensor 68 transitions from on to off can be accurately detected.

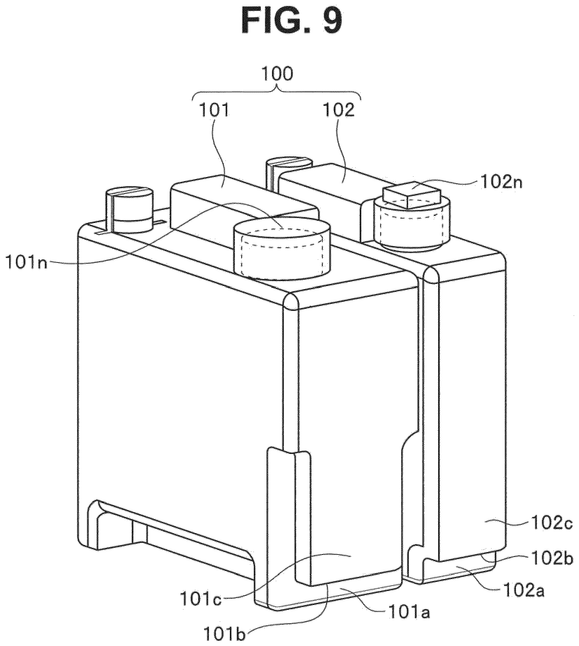

Fig. 9 is a perspective view showing the bottle 100 from a rear surface side.

A rear surface of the first bottle 101 is provided with a first inner wall surface 101a which corresponds to the first inner wall surface 100a, a stepped inner wall surface 101b which corresponds to the stepped inner wall surface 100b, and a second inner wall surface 101c which corresponds to the second inner wall surface 100c.

A rear surface of the second bottle 102 is provided with a first inner wall surface 102a which corresponds to the first inner wall surface 100a, a stepped inner wall surface 102b which corresponds to the stepped inner wall surface 100b, and a second inner wall surface 102c which corresponds to the second inner wall surface 100c.

As described earlier, since an amount of the first medicinal solution and an amount of the second medicinal solution which are used in reprocessing of one step generally differ from each other, a height of the stepped inner wall surface 101b from a bottom surface of the first bottle 101 and a height of the stepped inner wall surface 102b from a bottom surface of the second bottle 102 may differ from each other.

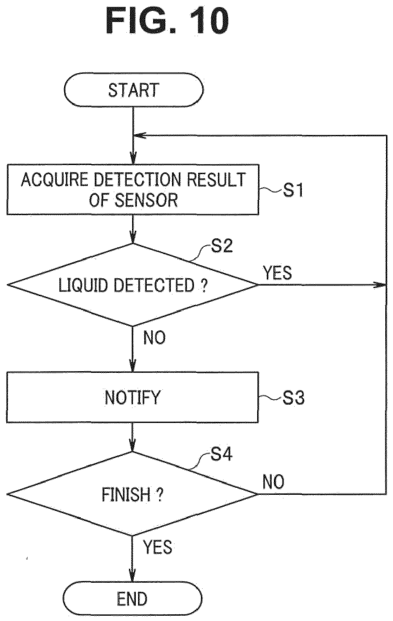

Fig. 10 is a flow chart showing an action by the processor 70 controlling a notification device to perform notification based on a detection result of the sensor 68.

As described above, the processor 70 is connected to respective circuits inside the endoscope reprocessor 1 including the sensor 68, the main operation panel 25, and the sub operation panel 13.

When power of the endoscope reprocessor 1 is turned on, the processor 70 performs processing shown in Fig. 10.

The processor 70 acquires a detection result from the sensor 68 (step S1) and determines whether or not the medicinal solution 103 is detected (step S2).

At this point, a determination that the medicinal solution 103 is detected means that the bottle 100 containing the medicinal solution 103 which enables n-number or more steps of reprocessing of the endoscope 200 to be performed is properly housed in the tray 12 and that the tray 12 is properly closed. In this case, a return is made to step S1 for each loop period constituted of step S1 and step S2 to acquire a detection result from the sensor 68.

In step S2, when the processor 70 determines that the medicinal solution 103 is not detected, the processor 70 performs a notification display on the main operation panel 25 or the sub operation panel 13 (step S3).

For example, when the notification display is performed immediately after replacing the bottle 100 and closing the tray 12, the user can recheck each state in consideration of any of possibilities that the tray 12 may not be properly closed, the bottle 100 may not be properly housed in the tray 12, and the bottle 100 housed in the tray 12 is not a bottle containing the correct medicinal solution 103.

In addition, with respect to the bottle 100 in use, when the detection result transitions from a state (on) where the medicinal solution 103 is detected to a state (off) where the medicinal solution 103 is not detected, the processor 70 determines that the remaining number of steps of the reprocessing of the endoscope 200 which can be performed is n-number of steps. When the processor 70 determines that the remaining number of steps of the reprocessing of the endoscope 200 which can be performed is n-number of steps, the processor 70 controls the main operation panel 25 or the sub operation panel 13 so as to notify that the remaining number of steps of the reprocessing which can be performed is n-number of steps.

At this point, an example of a notification display when n is 1 or more is "The remaining number of steps of reprocessing of the endoscope is n". In particular, when n is 1, a notification display such as "The next step of reprocessing of the endoscope will be the last" may be performed. In addition, an example of a notification display when n is 0 is "The medicinal solution in the bottle has run out. Please replace the bottle". Notification displays are not limited to these examples and an appropriate notification display may be performed.

While an example of performing a notification display using a display device such as the sub operation panel 13 or the main operation panel 25 has been described, an audio device such as a speaker may be used as the notification device to perform a notification by a buzzer or voice or to perform a notification by display and voice.

After performing a notification in step S3, a determination is made on whether or not processing is to be finished (step S4). When a determination is made at this point not to finish the processing, a return is made to step S 1 to acquire a detection result from the sensor 68.

Therefore, for example, when the notification display in step S3 is performed immediately after replacing the bottle 100, by returning to step S 1 and acquiring a detection result from the sensor 68, whether or not the bottle 100 containing the correct medicinal solution 103 is properly housed in the tray 12 and the tray 12 is properly closed to the reprocessor body 2 can be checked based on the detection result.

On the other hand, when a determination is made to finish the processing in step S4, the processing ends.

While an example of setting the bottle 100 to the endoscope reprocessor 1 by housing the bottle 100 in the tray 12 and opening or closing the tray 12 has been described above, the tray 12 need not be used in order to set the bottle 100. For example, a configuration may be adopted in which the endoscope reprocessor 1 is provided with a depressed shape portion for housing the bottle 100 and the bottle 100 is directly set to the endoscope reprocessor 1. Even in this case, the positional relationship between the respective inner wall surfaces of the bottle 100 in the set state and the sensor 68 need only be as described above.

In addition, while an example of detecting a remaining amount of the medicinal solution 103 contained in the bottle 100 with the sensor 68 has been described above, the detection of the remaining amount is not limited to the sensor 68 and a remaining amount of other liquids may be detected by a sensor. For example, a remaining amount of a cleaning agent inside the cleanser tank 11a may be detected by a sensor or a remaining amount of alcohol inside the alcohol tank 1 1b may be detected by a sensor. In this manner, a liquid to be an object of detection of a remaining amount by a sensor is not limited to the medicinal solution 103.

According to the first embodiment described above, since the sensor 68 is arranged so that the first inner wall surface 100a is outside of the detection range 68s and the stepped inner wall surface 100b is inside the detection range 68s in a state where the bottle 100 is set, even if relative positions of the bottle 100 and the sensor 68 deviate within a permissible range due to a size of the detection range 68s, the remaining amount of the medicinal solution 103 inside the bottle 100 being a predetermined amount can be accurately detected.

Since a configuration is adopted so that the bottle 100 is housed in the tray 12 and the sensor 68 is arranged so that the stepped inner wall surface 100b is inside the detection range 68s in a state where the tray 12 is closed, a determination that the tray 12 is properly closed can be made if the medicinal solution 103 is detected by the sensor 68. Therefore, the sensor 68 can also serve as a sensor for detecting an open/closed state of the tray 12.

Since the stepped inner wall surface 100b is provided at a height position where the medicinal solution 103 in an amount that enables n or more and less than (n+1) number of steps of reprocessing of the endoscope 200 to be performed remains inside the bottle 100, the fact that the remaining number of steps of the reprocessing of the endoscope 200 which can be performed is n-number of steps can be reliably determined.

Furthermore, since a notification is to be made when it is determined that the remaining number of steps of the reprocessing of the endoscope 200 which can be performed is n-number of steps, the user can accurately recognize a time for replacement of the bottle 100. Therefore, the user can perform preparation for replacing the bottle 100 in an efficient manner.

When the medical equipment is the endoscope 200, the endoscope reprocessor 1 suitable for reprocessing of the endoscope 200 can be obtained as the reprocessor for medical equipment.

As described above, a remaining amount of the medicinal solution 103 which does not enable a next reprocessing step to be guaranteed and which is created when the remaining amount of the medicinal solution 103 inside the bottle 100 cannot be accurately detected can be reduced. As a result, wasteful disposal of the medicinal solution 103 can be suppressed.

### [Second embodiment]

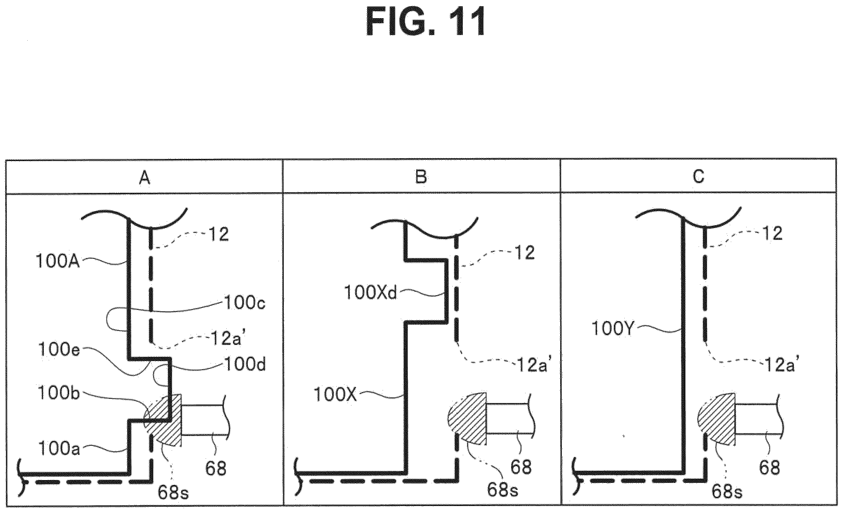

Fig. 11 is a chart which represents a second embodiment of the present invention and which shows a configuration example in which the stepped inner wall surface 100b is an inner wall surface on a lower side of a convex wall 100d provided at a predetermined height position of a bottle 100A in comparison to other configuration examples.

In the second embodiment, portions similar to the first embodiment described above will be denoted by same reference signs or the like and descriptions of such portions will be omitted as appropriate, and only differences will be mainly described.

The bottle 100A according to the present embodiment is provided with a projecting convex wall 100d. The convex wall 100d projects from between the first inner wall surface 100a on a lower side and the second inner wall surface 100c on an upper side in a state where the bottle 100A is set to the endoscope reprocessor 1.

The stepped inner wall surface 100b is an inner wall surface on a lower side of the convex wall 100d and is connected to the first inner wall surface 100a. In addition, an inner wall surface 100e on an upper side of the convex wall 100d is connected to the second inner wall surface 100c.

In addition, the second inner wall surface 100c is arranged in the direction of gravitational force G at, for example, a same position in the horizontal direction as the first inner wall surface 100a. In this arrangement, the second inner wall surface 100c is outside of the detection range 68s of the sensor 68.

In the configuration according to the present embodiment, a sensor hole 12a' provided in the tray 12 also serves as a hole into which the convex wall 100d is fitted.

Field A in Fig. 11 shows a state where the bottle 100A containing the correct medicinal solution 103 (not illustrated) is housed in the tray 12 and the tray 12 is closed.

The bottle 100A containing the correct medicinal solution 103 is provided with the convex wall 100d at a position corresponding to the sensor hole 12a'. When the tray 12 is closed, the convex wall 100d is fitted into the sensor hole 12a' and the stepped inner wall surface 100b enters the detection range 68s of the sensor 68. As a result, the sensor 68 changes from off to on and the processor 70 can determine that the correct bottle 100A containing the correct medicinal solution 103 has been properly set to the endoscope reprocessor 1.

Field B in Fig. 11 shows a state where a bottle 100X containing a liquid (not illustrated) which differs from the correct medicinal solution 103 is housed in the tray 12 and the tray 12 is closed.

The bottle 100X containing a liquid that differs from the correct medicinal solution 103 is provided with a convex wall 100Xd at, for example, a position that differs from the bottle 100A containing the correct medicinal solution 103. The bottle 100X with a shape that differs from the bottle 100A may be unhouseable in the tray 12. Even assuming that the bottle 100X can be housed in the tray 12 and the tray 12 can be closed, the convex wall 100Xd is not at a position where the convex wall 100Xd fits with the sensor hole 12a'.

Therefore, the liquid inside the bottle 100X does not enter the detection range 68s of the sensor 68 and the processor 70 cannot determine that the bottle 100X has been set to the endoscope reprocessor 1.

As described above, by differentiating a shape of a bottle in accordance with a type of liquid contained inside and providing the sensor hole 12a' of the tray 12 and the sensor 68 at positions in accordance with the bottle to be housed, a liquid that differs from the correct medicinal solution 103 inside the bottle 100X can be prevented from being used in reprocessing of the endoscope 200.

Field C in Fig. 11 shows a state where a bottle 100Y containing a liquid (not illustrated) which differs from the correct medicinal solution 103 is housed in the tray 12 and the tray 12 is closed.

The bottle 100Y containing a liquid that differs from the correct medicinal solution 103 is not provided with the convex wall 100d which is, for example, similar to the convex wall 100d of the bottle 100A containing the correct medicinal solution 103. As long as overall dimensions are substantially similar, such a bottle 100Y can be housed in the tray 12 and the tray 12 can be closed.

However, the liquid inside the bottle 100Y does not enter the detection range 68s of the sensor 68 and the processor 70 cannot determine that the bottle 100Y has been set to the endoscope reprocessor 1.

As described above, even when the bottle 100Y containing a liquid that differs from the correct medicinal solution 103 is not provided with the convex wall 100d, the liquid inside the bottle 100Y can be prevented from being used in reprocessing of the endoscope 200.

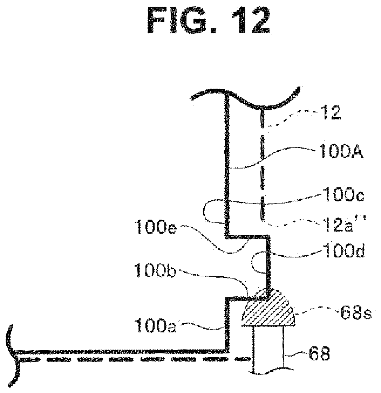

Fig. 12 is a diagram showing an arrangement of the sensor 68 according to a modification of the second embodiment.

In the example shown in Fig. 11, the sensor 68 is arranged in the horizontal direction and the detection range 68s is in the horizontal direction of the sensor 68. In contrast, Fig. 12 shows the sensor 68 being arranged upward in a vertical direction and the detection range 68s being on an upper side of the sensor 68. A sensor hole 12a" of the tray 12 is formed in a shape that accepts the sensor 68 arranged in the vertical direction.

Even with such an arrangement, an operation and an effect similar to the operation and the effect described above may be produced as long as the stepped inner wall surface 100b is inside the detection range 68s and the first inner wall surface 100a is outside of the detection range 68s.

Furthermore, the arrangement of the sensor 68 is not limited to the horizontal direction and the vertical direction and the sensor 68 may be arranged in an oblique direction. Such an arrangement of the sensor 68 in the vertical direction or the oblique direction can be similarly applied to the configuration of the first embodiment described above.

According to the second embodiment described above, in addition to producing a substantially similar effect to the first embodiment described above, since the bottle 100A is provided with the convex wall 100d and the stepped inner wall surface 100b is made an inner wall surface on a lower side of the convex wall 100d, the fact that the bottle 100A containing the correct medicinal solution 103 has been properly set to the endoscope reprocessor 1 can be determined based on a detection result of the sensor 68 due to the convex wall 100d fitting with the sensor hole 12a of the tray 12. As a result, a liquid that differs from the correct medicinal solution 103 can be prevented from being used in reprocessing of the endoscope 200.

Note that the present invention is not limited to the above-described embodiments as it is, and can be embodied by modifying constituent elements without departing from the scope of the invention in an implementation stage. In addition, various aspects of the invention can be formed by appropriately combining a plurality of constituent elements disclosed in the embodiments described above. For example, some constituent elements may be deleted from all the constituent elements shown in the embodiments. Furthermore, constituent elements over different embodiments may be appropriately combined. In this manner, various modifications and applications of the present invention can obviously be made without departing from the spirit of the invention.

## Claims

1. A reprocessor for medical equipment to which a bottle containing a liquid for reprocessing medical equipment is set, the reprocessor for medical equipment comprising:
a sensor configured to detect that the liquid is within a detection range, wherein
the sensor is arranged such that, in a state where the bottle is set to the reprocessor for medical equipment, a first inner wall surface of the bottle arranged in a direction of gravitational force is outside of the detection range and a stepped inner wall surface connected to an upper side of the first inner wall surface at an angle intersecting the direction of gravitational force is inside the detection range.

2. The reprocessor for medical equipment according to claim 1, further comprising:
a reprocessor body; and
a tray which is openable and closable with respect to the reprocessor body and which is configured to house the bottle, wherein
the sensor is arranged in the reprocessor body so that the stepped inner wall surface is inside the detection range in a state where the tray is closed.

3. The reprocessor for medical equipment according to claim 1, further comprising:
a processor to which a detection result of the sensor is inputted, wherein
the bottle contains the liquid in an amount to be used in a plurality of steps of reprocessing of the medical equipment in an unused state,
when n denotes an integer equal to or larger than 0, the stepped inner wall surface is provided at a height position where the liquid in an amount that enables n or more and less than (n+1) number of steps of the reprocessing to be performed remains inside the bottle at a time point where the liquid transitions from a state of being in contact with the stepped inner wall surface to a state of not being in contact with the stepped inner wall surface in a state where the bottle is set to the reprocessor for medical equipment, and
the processor is configured to determine that a remaining number of steps of the reprocessing of the medical equipment which can be performed is n-number of steps when the detection result transitions from a state where the liquid is detected to a state where the liquid is not detected.

4. The reprocessor for medical equipment according to claim 3, further comprising:
a notification device configured to notify a user, wherein
when the processor determines that the remaining number of steps of the reprocessing of the medical equipment which can be performed is n-number of steps, the processor is configured to control the notification device so as to notify that the remaining number of steps of the reprocessing which can be performed is n-number of steps.

5. The reprocessor for medical equipment according to claim 1, further comprising:
a cleaning tank configured to house the medical equipment and to reprocess the medical equipment with the liquid.

6. The reprocessor for medical equipment according to claim 1, wherein the medical equipment is an endoscope.

7. A bottle which contains a liquid for reprocessing medical equipment and which is to be set to a reprocessor for medical equipment including a sensor configured to detect that the liquid is within a detection range, the bottle comprising:
a first inner wall surface; and
a stepped inner wall surface connected to the first inner wall surface, wherein
in a state where the bottle is set to the reprocessor for medical equipment,
the first inner wall surface is arranged in a direction of gravitational force and is outside of the detection range, and
the stepped inner wall surface is connected to an upper side of the first inner wall surface at an angle intersecting the direction of gravitational force and is inside of the detection range.

8. The bottle according to claim 7, further comprising:
a second inner wall surface arranged in the direction of gravitational force at a different horizontal direction position from the first inner wall surface in a state where the bottle is set to the reprocessor for medical equipment, wherein
the stepped inner wall surface is a surface which connects the first inner wall surface arranged on a lower side and the second inner wall surface arranged on an upper side.

9. The bottle according to claim 7, further comprising,
in a state where the bottle is set to the reprocessor for medical equipment:
a second inner wall surface which is arranged in the direction of gravitational force and which is outside of the detection range; and
a convex wall which is provided between the first inner wall surface on a lower side and the second inner wall surface on an upper side and which is configured to project from the first inner wall surface and the second inner wall surface, wherein
the stepped inner wall surface is an inner wall surface on a lower side of the convex wall.

10. A reprocessor for medical equipment to which a bottle containing a liquid for reprocessing medical equipment is set, the reprocessor for medical equipment comprising:
a sensor configured to detect that the liquid is within a detection range, wherein
in a state where the bottle is set to the reprocessor for medical equipment, the sensor is configured to detect the liquid when a liquid level of the liquid is present above a step provided on a side surface of the bottle but configured not to detect the liquid when the liquid level of the liquid is present below the step.

11. The reprocessor for medical equipment according to claim 10, further comprising:
a cleaning tank configured to house the medical equipment and to reprocess the medical equipment with the liquid.
